# EUROPÄISCHE PATENTSCHRIFT

(11) **EP 1 287 785 B1**
(45) Veröffentlichungstag und Bekanntmachung des Hinweises auf die Patenterteilung: **13.12.2006**
(21) Anmeldenummer: 02018994.0
(22) Anmeldetag: 27.08.2002
(51) Int. Cl.: A61B 5/15

(54) **Analytisches Hilfsmittel mit Lanzette und Testelement**
Analyzing means with lancet and test element
Moyen d'analyse avec lancette et élément d'essai

(30) Priorität: 29.08.2001 DE 10142232
(43) Veröffentlichungstag der Anmeldung: 05.03.2003
(60) Teilanmeldung: 04016622.5
(73) Patentinhaber: Roche Diagnostics GmbH, 68305 Mannheim (DE); F.HOFFMANN-LA ROCHE AG, 4070 Basel (CH)
(72) Erfinder: Kuhr, Hans-Juergen, Dr., 68219 Mannheim (DE); Fritz, Michael, 68647 Bilbis (DE); Weiss, Thomas, 68307 Mannheim (DE); Forster, Richard, 92536 Pfreimd (DE)

(56) Entgegenhaltungen:
- WO-A1-01/66010
- DE-B1- 2 803 345
- US-A- 5 636 640
- US-A- 6 036 924
- US-A- 6 143 164

## Beschreibung

Die Erfindung betrifft ein analytisches Hilfsmittel, welches eine Lanzette und ein analytisches Testelement enthält.

Die Untersuchung von Blutproben ermöglicht in der klinischen Diagnostik das frühzeitige und zuverlässige Erkennen von pathologischen Zuständen sowie die gezielte und fundierte Kontrolle von Körperzuständen. Die medizinische Blutdiagnostik setzt stets die Gewinnung einer Blutprobe des zu untersuchenden Individuums voraus. Während in Kliniken und bei niedergelassenen Ärzten oftmals durch eine Venenpunktion mehrere Milliliter Blut einer zu untersuchenden Person für die Analyse gewonnen werden, um damit eine Vielzahl von Labortests durchführen zu lassen, reichen für einzelne Analysen, die gezielt auf einen Parameter gerichtet sind, heutzutage oftmals wenige Mikroliter bis hin zu weniger als einem Mikroliter Blut aus. Solch geringe Blutmengen erfordern keine aufwendige und schmerzhafte Venenpunktion. Vielmehr genügt es hier, zur Blutgewinnung durch die Haut z. B. in die Fingerbeere oder das Ohrläppchen der zu untersuchenden Person mit Hilfe einer sterilen, scharfen Lanzette zu stoßen, um so einige wenige Mikroliter Blut oder weniger für die Analyse zu gewinnen. Insbesondere eignet sich diese Methode, wenn die Analyse der Blutprobe unmittelbar nach der Blutgewinnung durchgeführt werden kann.

Vor allem im Bereich des sogenannten "Home-Monitoring", also dort, wo medizinische Laien selbst einfache Analysen des Bluts durchführen, und dort insbesondere für die regelmäßige, mehrmals täglich durchzuführende Blutgewinnung durch Diabetiker für die Kontrolle der Blutglucosekonzentration, werden Lanzetten und dazu passende Geräte (sogenannte Blutentnahmegeräte, Blutlanzettenvorrichtungen oder - wie sie im Folgenden genannt werden sollen - Stechhilfen), angeboten, die eine möglichst schmerzarme und reproduzierbare Blutgewinnung ermöglichen. Zudem soll die Verwendung von Lanzetten mit Stechhilfen die psychologische Schwelle beim Stechen des eigenen Körpers senken, was vor allem für Kinder, die an Diabetes erkrankt sind und aufregelmäßige Blutglucosetests angewiesen sind, von besonderer Bedeutung ist. Als Beispiele für Lanzetten und Stechhilfen seien die kommerziell erhältlichen Geräte (Stechhilfen) und Lanzetten Glucolet^{®} der Bayer AG und Sofidix^{®} der Roche Diagnostics GmbH genannt. Solche Lanzetten und Geräte (Stechhilfen) sind z. B. Gegenstand von WO-A 98/48695, EP-A 0 565 970, US 4,442,836 oder US 5,554,166.

Die eigenhändige Blutzuckerbestimmung (das sogenannte "Home Monitoring") ist heute eine weltweit verbreitete Methode in der Diabetes-Kontrolle. Blutzuckergeräte im Stand der Technik wie z. B. Accu-Chek Sensor (von Roche Diagnostics) bestehen aus einem Messgerät, in das ein Testelement (Teststreifen, Sensor) eingeführt wird. Der Teststreifen wird mit einem Blutstropfen in Kontakt gebracht, der zuvor mittels einer Stechhilfe aus der Fingerbeere gewonnen wurde. Die zahlreichen Systemkomponenten (Lanzette, Stechhilfe, Teststreifen und Messgerät) benötigen viel Platz und bedingen ein relativ komplexes Handling. Inzwischen gibt es auch Systeme mit einem höheren Grad der Integration und damit einer einfacheren Handhabung. Hierzu gehören beispielsweise das AccuChek Compact (von Roche Diagnostics), das Glucometer Dex (von Bayer Diagnostics) sowie das Soft-Sense (von Medisense). Bei den beiden erstgenannten Systemen werden die Teststreifen im Messgerät magaziniert und für die Messung zur Verfügung gestellt.

Ein nächster Schritt der Miniaturisierung ist beispielsweise durch die Integration von mehreren Funktionen bzw. Funktionselementen in einem einzigen analytischen Hilfsmittel (Disposable) zu erreichen. Durch geeignete Kombination von Stechvorgang und sensorischer Analytkonzentrations-Erfassung auf einem Teststreifen lässt sich beispielsweise der Bedienablauf deutlich vereinfachen. Hierfür gibt es im Stand der Technik folgende Beispiele:

EP-B 0 199 484 (Audio Bionics) beschreibt ein analytisches Hilfsmittel ("Disposable"; kurz: Dispo) mit integrierter Lanzette, die geräteseitig aktuiert wird (siehe zum Beispiel Figur 9). Mittels einer spezifischen Federlagerung (,spring-mounted lance means') wird die Lanzette nach dem Einstich wieder zurückgezogen. Das Dispo enthält ein sogenanntes Docht-Mittel ("wick means") durch das die Probenflüssigkeit von der Körperoberfläche zum Analysebereich, einem optisch auswertbaren Testfeld, geleitet wird.

In US 6,143,164 (E. Heller & Comp.) wird ein Verfahren beschrieben, bei dem eine Körperöffnung (beispielsweise ein kleiner Einstich oder Schnitt durch die Haut) erzeugt und anschließend Körperflüssigkeit in einen Sensor transportiert und dort auf die Gegenwart eines Analyten untersucht wird. US 6,143,164 offenbart hierzu ein analytisches Hilfsmittel, bei dem eine Lanzettenvorrichtung auf einem Sensor-Teststreifen angebracht ist. Der Transport der Probenflüssigkeit von der Körperöffnung zum eigentlichen Nachweiselement des Sensors erfolgt beispielsweise wieder über ein "wick means" oder auch über Kapillarspalte/-kanäle.

WO 99/26539 (Mercury Diagnostics), US 5,951,492 (Mercury Diagnostics) und US 6,056,701 (Amira Medical) beschreiben unter anderem Sammeldevices für Körperflüssigkeiten mit einem länglichen Stiel, an dessen Kopfbereich ein Testfeld angebracht ist.

US 6,032,059 (Abbott) und US 6,014,577 (Abbott) beschreiben ein Disposable mit einer Kanüle, die zum Einstich in die Haut verwendet wird. Durch einen in die Kanüle integrierten Sensor wird Körperflüssigkeit analysiert, die zuvor mittels Kapillarwirkung in die Kanüle eingesaugt wurde.

US 5,801,057 (Smart et al.) beschreibt ein aus Silizium gefertigtes Disposable mit einer integrierten Hohlnadel. Am Ende dieser Hohlnadel befindet sich eine Sammelkammer für eingesogene Körperflüssigkeit. Durch eine geeignete Reaktionschemie kann daraus die Konzentration eines Blutbestandteils bestimmt werden.

US 5,035,704 (Lambert et al.) offenbart ein System zur Blutgewinnung mit einem Magazin aus Disposables. Ein Lanzettenelement kann dabei in den Dispos integriert sein. Die Übertragung eines Blutstropfens auf das Testfeld geschieht durch direkten Hautkontakt und kann durch ein angelegtes Vakuum verstärkt werden.

US 6,132,449 (Agilent Technologies) offenbart ein integriertes Dispo mit Stech- und Messfunktion. Das stechende Element wird senkrecht zur Dispo-Ebene aktiviert und tritt dabei durch das Dispo hindurch. Die Wundöffnung ist in direktem Kontakt mit mehreren Kapillarstrukturen, die das austretende Blut in einen separaten Analyseteil des Dispos transportieren.

Das Dokument WO 01/66010 beschreibt eine Lanzettennadel, bei der die Spitze entweder in ein elastisches Material eingebettet ist oder sich in einem Hohlkörper befindet von dem sie umgeben ist. Auf diese Weise wird erreicht, dass die Lanzettennadel vor dem Einstich steril gehalten wird. Beim Stechvorgang durchsticht die Lanzettenspitze das elastische Material beziehungsweise den Hohlkörper und tritt aus. Dieses Dokument wurde veröffentlich am 13.09-2001 und fällt daher unter Artikel 54(3) EPÜ.

Ein zentrales Problem der Blutgewinnung mit einem sogenannten "integrierten Disposable" (bei dem Lanzette und Testelement miteinander verbunden sind bzw. einen Einheit bilden) ist die Tatsache, dass nach einem Einstich das Kapillarblut meist nicht selbsttätig aus der Wunde austritt. Durch direkten Kontakt eines Disposables mit der Wundöffnung wird dieser negative Effekt noch verstärkt. Nach einem Einstich muss der Blutstropfen aktiv an die Hautoberfläche befördert werden, indem man beispielsweise die Wunde mechanisch öffnet und/oder einen leichten Druck auf das Gewebe rund um den Wundbereich ausübt (beispielsweise durch einfaches "Fingermelken"). Das Anlegen von Vakuum kann diesen Vorgang sinnvoll unterstützen.

Wie Untersuchungen im Labor gezeigt haben, ist es vorteilhaft, dass die Wundöffnung für den Zeitraum der Blutgewinnung freibleibt. Diese Tatsache bedingt jedoch einen komplizierten Bewegungsablauf beim Dispo, da eine sehr schnelle Stechbewegung und eine langsame Blutaufnahmebewegung mit einer Wartezeit im System zu realisieren sind. Hierzu sind nur wenige der im Stand der Technik vorgeschlagenen Dispo-Konzepte geeignet.

Insbesondere eine starre, herausragende Lanzettenspitze kann beim Aufnehmen des Blutstropfens zu ungewollten Verletzungen führen. Eine relativ zum Disposable bewegliche Stechvorrichtung ist somit zu bevorzugen. Diese Anforderung in Kombination mit den im Stand der Technik bekannten Dispos führt jedoch zu einer aufwendigen und damit auch teuren Dispoausführung.

Ein weiteres Manko bekannter Dispos ist die Sicherstellung der Lanzettensterilität für den Zeitraum der Aufbrauchfrist. Im Stand der Technik bekannt ist der Spitzenschutz durch eine Kappe, die vor Gebrauch manuell entfernt wird. Die Automatisierung von Blutzuckermessungen wird jedoch durch solch eine Kappe erschwert. Bisher beschriebene Konzepte, die eine automatische Blutzuckerbestimmung mit einem integrierten Disposable prinzipiell ermöglichen würden, weisen den Nachteil auf, dass eine latente Verschmutzungsgefahr der Lanzettennadelspitze vorhanden ist. Bestandteile des Testfeldes (Chemiekalien, biologische Bestandteile, Klebstoffe etc.) können durch die Luft oder über Oberflächen innerhalb des Dispos wandern. Eine anfänglich durchgeführte Sterilisation der Nadelspitze ohne weitere schützende Maßnahmen ist somit keinesfalls ausreichend, um die Anforderungen an ein steriles Medizinprodukt einzuhalten.

Die Lanzetten des Standes der Technik weisen meist eine metallene Lanzettennadel mit einer Spitze auf, die gegebenenfalls angeschliffen sein kann. Zur leichteren Handhabung der Lanzette und gegebenenfalls zu ihrer Befestigung in einer Stechhilfe ist bei vielen Ausführungsformen meist an die Lanzettennadel ein Kunststofflanzettenkörper aus einem starren, spritzgußfähigen Material angespritzt. Die Spitze der Lanzettennadel ist - im unbenutzten Zustand - zur Sicherstellung ihrer Sterilität von einer Schutzhülle umgeben. Diese besteht in der Regel aus dem selben starren Material wie der eigentliche Lanzettenkörper und bildet meist mit diesem eine Einheit. Die Schutzhülle kann vor der Benutzung der Lanzette vom Lanzettenkörper getrennt und von der Spitze der Lanzettennadel abgenommen werden. Zwischen Lanzettenkörper und Schutzhülle befindet sich zu diesem Zweck meist eine Sollbruchstelle. Nach der Benutzung der Lanzette liegt die Spitze der Lanzettennadel ungeschützt vor und stellt somit eine potentielle Verletzungsquelle für den Benutzer und eventuell andere Personen dar.

Die Aufgabe der vorliegenden Erfindung war es daher, die Nachteile des Standes der Technik, insbesondere die eingangs erwähnten Nachteile zu beseitigen. Insbesondere war es die Aufgabe der vorliegenden Erfindung, analytische Hilfsmittel (oder synonym dazu: "Disposables", kurz: "Dispos") zur Verfügung zu stellen, die die Nachteile des Standes der Technik nicht zeigen. Ganz besonders sollte die Sicherstellung der Lanzettensterilität für den Zeitraum der Aufbrauchfrist durch das erfindungsgemäße Dispo bei gleichzeitiger Integration von Lanzette und Testelement (Teststreifen, Sensor) gewährt werden. Selbstverständlich sollte ebenfalls ein Herstellverfahren für solch ein analytisches Hilfsmittel beschrieben werden. Weiterhin ist es die Aufgabe der vorliegenden Erfindung, analytische Hilfsmittel mit Lanzetten bereitzustellen, bei denen zumindest die Lanzettennadelspitze im unbenutzten Zustand bis unmittelbar vor der Benutzung steril, das heißt keimfrei, gehalten wird und im benutzten Zustand hygienisch aufbewahrt werden kann. Idealerweise sollte diese Aufgabe gelöst werden, ohne daß der Benutzer für die hygienische Aufbewahrung separate Maßnahmen zu ergreifen hat. Zudem sollte der Benutzer vor unbeabsichtigter Verletzung mit der Lanzette, insbesondere der benutzten Lanzette geschützt sein. Schließlich sollte vorzugsweise ein einfacher Probentransfer von der Stelle der Blutgewinnung zur Stelle der Blutuntersuchung möglich sein.

Diese Aufgabe wird durch den Gegenstand der Erfindung, wie er in den unabhängigen Patentansprüche charakterisiert wird, gelöst. Bevorzugte Ausführungsformen sind Gegenstand der abhängigen Ansprüche.

Ein erster Gegenstand der Erfindung ist ein analytisches Hilfsmittel, das eine Lanzette enthält. Die Lanzette weist als wesentliche Bestandteile eine Lanzettennadel mit einer Spitze und einen Lanzettenkörper auf, der die Lanzettennadel zumindest im Bereich der Spitze vollständig umgibt. Die Lanzettennadel ist dabei relativ zum Lanzettenkörper verschiebbar. Der Lanzettenkörper besteht zumindest im Bereich der Spitze der Lanzettennadel aus einem elastischen Material, in das die Spitze der Lanzettennadel eingebettet ist. Das analytische Hilfsmittel enthält weiterhin ein analytisches Testelement, das fest mit dem Lanzettenkörper verbunden ist.

Ein weiterer Gegenstand der Erfindung ist ein weiteres analytisches Hilfsmittel, das eine Lanzette enthält. Die Lanzette umfasst dabei eine Lanzettennadel mit einer Spitze und einen Lanzettenkörper, der im Bereich der Spitze der Lanzettennadel als Hohlkörper ausgebildet ist und der die Spitze der Lanzettennadel umgibt. Die Lanzettennadel ist auch hier relativ zum Lanzettenkörper verschiebbar. Der Hohlkörper besteht zumindest teilweise aus einem elastischen Material, das von der Spitze der Lanzettennadel beim Stechvorgang durchstoßen werden kann und das sich gegebenenfalls nach dem Zurückziehen der Spitze der Lanzettennadel in den Hohlkörper wieder verschließt. Das analytische Hilfsmittel enthält weiterhin ein analytisches Testelement, das fest mit dem Lanzettenkörper verbunden ist.

Die erfindungsgemäße Lösung besteht vorzugsweise aus einem miniaturisierten Dispo, bei dem die drei Funktionen Stechen, Bluttransfer von der durch das Stechen erzeugten Wunde zum Testelement, und Sensorik in einem Teil zusammengefasst sind.

Der Grundkörper des erfindungsgemäßen analytischen Hilfsmittels besteht aus einem starren Kunststoffkörper, dessen äußere Form vorzugsweise zum Zwecke der Halterung in einem Gerät entsprechend angepasst ist. In diesem Kunststoffwird eine Lanzettennadel derart eingebettet, dass ihre Spitze vorzugsweise nicht über die Vorderkante des Grundkörpers herausragt. Der Grundkörper kann daher auch als Lanzettenkörper bezeichnet werden. Der Grundköper besitzt in einer bevorzugten Ausführungsform Stege, die zur Fixierung der Nadel im Grundkörper und zur Führung während der Stechbewegung dienen.Vorzugsweise ist der überwiegende Teil der Nadel jedoch nicht mit dem Grundkörper verbunden, um die Reibungskräfte bei der Stechbewegung zu minimieren. Bevorzugt werden die Kontaktflächen zwischen Nadel und Lanzettenkörper minimiert und geeignet vorbehandelt, beispielsweise silikonisiert.

Die erfindungsgemäßen Lanzetten sind vorzugsweise für den einmaligen Gebrauch konzipiert und können daher auch als Einweg-Blutlanzetten oder Wegwerf-Blutlanzetten bezeichnet werden. Die Lanzette der Erfindung beinhaltet eine Nadel (Lanzettennadel) mit einer Spitze. Die Nadel ist in der Regel mehrere Millimeter (mm) bis wenige Zentimeter (cm) lang und weist eine längliche Gestalt auf. Typischerweise besitzen Nadeln eine zylindrische Gestalt, da diese Nadelform besonders gut herstellbar ist; es sind jedoch auch Nadelformen mit abweichender Formgebung möglich. Der Spitzenbereich der Nadel beinhaltet die Nadelspitze, die beim bestimmungsgemäßen Gebrauch der Lanzette in Gewebe eingestochen wird. Die Spitze der Lanzettennadel ist folglich der Teil der Lanzette, der mit der Haut des zu stechenden Individuums in Berührung kommt, diese ggf. verletzt und so den Ausfluß einer Körperflüssigkeit, insbesondere Blut oder interstitieller Flüssigkeit, verursacht.

Die Spitze der Lanzettennadel kann beispielsweise rotationssymmetrisch sein, wie dies im Allgemeinen bei Stecknadeln der Fall ist. Es hat sich jedoch als vorteilhaft herausgestellt, wenn man an der Nadelspitze einen oder mehrere Schliffe anbringt. Die hierbei entstehenden, zur Längsachse der Nadel geneigten, in einer Spitze zulaufenden Kanten dienen beim Einstich als scharfe Schneide und gestalten den Einstichvorgang schmerzärmer, als dies mit rotationssymmetrischen Nadeln der Fall ist.

Die Lanzettennadel der erfindungsgemäßen Lanzette ist aus einem Material gefertigt, das ausreichend hart ist, um eine mechanische Beanspruchung während des Einstichvorgangs, der Bearbeitungsschritte oder eventuell sonstige auftretende Beanspruchungen ohne Deformation zu überstehen. Weiterhin muß das Material so beschaffen sein, daß während des Einstichvorgangs keine Partikel abbrechen oder sich ablösen. Schließlich muß das Nadelmaterial auch so bearbeitbar sein, daß die Nadelspitze ausreichend spitz und die Kanten der Nadelspitze gegebenenfalls ausreichend scharf geschliffen werden können. Gut geeignete Materialien für die Lanzettennadel sind vor allem Metalle und von diesen insbesondere Edelstähle. Es sind jedoch auch Nadeln aus Silizium, Keramik oder Kunststoffen denkbar. Edelstahlnadeln sind besonders bevorzugt.

Erfindungsgemäß ist in einer Ausführungsform zumindest die Spitze der Lanzettennadel der erfindungsgemäßen Lanzette vom Lanzettenkörper umgeben. Wesentlich ist dabei, daß der Lanzettenkörper im Bereich der Spitze der Lanzettennadel aus einem elastischen Material besteht. Zumindest die Spitze der Lanzettennadel ist von diesem elastischen Material allseitig vollständig umgeben, das heißt in dieses eingebettet, und so von der Umgebung abgeschlossen. Das elastische Material des Lanzettenkörpers, welches in unterschiedlichen Ausführungsformen den Lanzettenkörper vollständig oder nur teilweise bilden kann, zeichnet sich dadurch aus, daß es weich, verformbar und von der Lanzettennadel mit ihrer Spitze durchstoßbar ist, ohne die Spitze zu verletzen. Beim Stechvorgang wird die Lanzettennadel entlang ihrer Längsachse relativ zum Lanzettenkörper bewegt und tritt mit ihrer Spitze aus dem Lanzettenkörper aus, um so zur Blutgewinnung in die Haut des zu untersuchenden Individuums einstechen zu können. Eine wichtige und erfindungsgemäß bevorzugte Eigenschaft ist ferner, daß sich das elastische Material gegebenenfalls beim Zurückziehen der Lanzettennadel in den Lanzettenkörper wieder dicht um die Spitze der Lanzettennadel verschließt. Nach dem Stechvorgang kann die Lanzettennadel in einer bevorzugten Ausführungsform in Umkehrung der Bewegung beim Stechvorgang in ihre Ausgangslage relativ zum Lanzettenkörper gebracht werden, in der die Spitze wieder allseitig vollständig vom elastischen Material des Lanzettenkörpers umschlossen ist.

Das elastische Material des Lanzettenkörpers, welches die Spitze der Lanzettennadel vollständig umschließt, gewährleistet die Sterilität der Lanzettennadelspitze vor deren Benutzung, vorzugsweise bis unmittelbar vor deren Benutzung, und sorgt gegebenenfalls für ein hygienisches Umschließen der Lanzettennadelspitze nach deren Benutzung. Der Begriff 'hygienisches Umschließen' ist so zu interpretieren, daß das unter Umständen nach dem Stich an der Nadel anhaftende biologische Material (Gewebe, Körperflüssigkeit) im Wesentlichen vom elastischen Material eingekapselt wird. Insbesondere Keime und infektiöses Material können somit nicht oder nur stark reduziert an die Außenwelt gelangen. Das elastische Material ist folglich keimdicht für das Eindringen oder Entweichen von Keimen, je nach dem, ob die Lanzettennadel unbenutzt oder benutzt ist. Zudem stellt das elastische Material einen mechanischen Schutz für die Lanzettennadelspitze dar und verhindert so auch ein unbeabsichtigtes Verletzen an der Lanzettennadelspitze.

Als elastisches Material für den Lanzettenkörper der vorliegenden Erfindung haben sich Gummi, Kautschuk, Silikon, Elastomere und insbesondere thermoplastische Elastomere als geeignet herausgestellt. Diese weisen die für die vorliegende Erfindung wesentlichen Eigenschaften auf: sie sind weich, verformbar, von der Lanzettennadel zu durchstoßen, ohne die Spitze zu verletzen, und schließen sich dicht um die benutzte Lanzettennadelspitze. Des weiteren können Sie für Spritzgußprozesse verwendet werden, die eine Massenfertigung von Lanzetten in großen Stückzahlen ermöglichen.

Thermoplastische Elastomere, die auch Elastoplaste oder Thermolaste oder thermoplastische Kautschuke genannt werden, besitzen im Idealfall eine Kombination der Gebrauchseigenschaften von Elastomeren und den Verarbeitungseigenschaften von Thermoplasten. Thermoplastische Elastomere sind beispielsweise Styrol-Oligoblock-Copolymere (sogenannte TPE-S), thermoplastische Polyolefine (TPE-O), thermoplastische Polyurethane (TPE-U), thermoplastische Copolyester (TPE-E) und thermoplastische Copolyamide (TPE-A). Insbesondere haben sich beispielsweise thermoplastische Elastomere auf der Basis von Styrol-Ethylen-Butylen-Styrol-Polymeren (SEBS-Polymere, z. B. Evoprene^{®} von Evode Plastics oder Thermolast K von Gummiwerk Kraiburg GmbH) als geeignet erwiesen.

Während des Stechvorgangs wird die Lanzettennadel relativ zum Lanzettenkörper bewegt. Letzterer wird dabei vorzugsweise von der Stechhilfe oder dem Stechgerät in seiner Position fixiert. Die Lanzettennadel kann zum Zwecke ihres Antriebs besonders ausgeformt sein, beispielsweise einen Nadelkopf an dem der Spitze entgegengesetzten Ende besitzen, oder zusätzlich zum Lanzettenkörper, der die Spitze umschließt, einen weiteren Lanzettenkörper aufweisen, der von einem Antriebselement der Stechhilfe ergriffen werden kann. Die Ausformung der Nadel oder der zusätzliche Lanzettenkörper können in geeigneter Weise mit einer entsprechenden Antriebsvorrichtung im Stechgerät (Stechhilfe) wechselwirken. Allgemein können solche Mittel als Verdickung der Nadel bezeichnet werden.

Zur Erzielung des Vorteils, daß die Lanzettennadelspitze vor Gebrauch steril vom elastischen Material des Lanzettenkörpers umschlossen ist, und nach ihrem Gebrauch hygienisch vom elastischen Material umgeben ist, ist es natürlich notwendig, die Lanzettennadel nach ihrem Gebrauch, d. h. nach dem Stechvorgang, weitgehend in ihre ursprüngliche relative Position bezüglich des Lanzettenkörpers enthaltend das elastische Material zu bringen. Dies kann durch geeignete Wechselwirkung mit einer entsprechend angepaßten Stechhilfe erreicht werden. Wichtig ist dabei nur, daß die Lanzettennadelspitze nach ihrer Benutzung wieder vom elastischen Material des Lanzettenkörpers umschlossen ist und so ein unbeabsichtigtes Verletzen an der Nadelspitze verhindert wird.

Zur Erhöhung der Stabilität des elastischen Materials ist es möglich, dieses mit einem steifen Material, beispielsweise einem steifen Kunststoffmaterial, zu verbinden. Das elastische Material kann dabei beispielsweise auf seiner Außenseite, die nicht mit der Spitze der Lanzettennadel in Berührung kommt, mit einer Schicht eines steifen Materials, beispielsweise eines steifen Kunststoffs, stabilisiert sein. Es ist auch möglich, den Lanzettenkörper nur im Bereich der Lanzettennadelspitze aus einem elastischen Material zu fertigen, im übrigen den Lanzettenkörper jedoch aus herkömmlichen, steifen Kunststoffen zu fertigen. Dabei können das elastische Material und das steife Material miteinander verklebt sein oder durch einen Spritzgußprozeß, beispielsweise einen Zweikomponenten-Spritzgußprozeß, miteinander verbunden sein. Das steife Material des Lanzettenkörpers sorgt dabei für eine mechanische Stabilisierung des elastischen Materials während des Stechvorgangs und erleichtert die Fixierung des elastischen Teils des Lanzettenkörpers während des Stechvorgangs durch die Stechhilfe. Das steife Material kann auch Teil des Testelements sein, beispielsweise eines Kapillarspalttestelements wie es in WO 99/29429 beschrieben ist.

In einer weiteren Ausführungsform der Erfindung enthält die Lanzette eine Lanzettennadel mit einer Spitze und einen Hohlkörper, der zumindest die Spitze der Lanzettennadel umgibt, wobei die Lanzettennadel im Bereich ihrer Spitze im Hohlkörper beweglich ist und der Hohlkörper zumindest teilweise aus einem elastischen Material besteht, das von der Spitze der Lanzettennadel beim Stechvorgang durchstoßen werden kann und das sich gegebenenfalls nach dem Zurückziehen der Spitze der Lanzettennadel in den Hohlkörper wieder verschließt.

Während bei der weiter oben beschriebenen Lanzette gemäß der ersten Ausführungsform die Lanzettennadel zur Gewährung der Sterilität vor Benutzung und der hygienischen Abgeschirmtheit nach der Benutzung im Bereich ihrer Spitze allseitig vollständig und somit ohne verbleibenden Hohlraum um die Spitze von einem elastischen Material umgeben ist, welches die Lanzettennadelspitze in sich einbettet, ist bei der jetzt beschriebenen zweiten Ausführungsform die Spitze der Lanzettennadel von einem allseits geschlossenen Hohlkörper umgeben. Vorteilhafterweise ist dieser Hohlkörper in den Bereichen, die nicht mit der Spitze der Lanzettennadel in Berührung kommen, aus einem steifen, vorzugsweise spritzgußfähigen Material gefertigt. Erfindungswesentlich ist, daß der Hohlkörper in dem Bereich, in dem er beim Stechvorgang von der Lanzettennadelspitze durchstoßen wird, aus einem elastischen Material besteht.

Beim Stechvorgang wird die Lanzettennadel relativ zum Hohlkörper, der den Lanzettenkörper darstellt, bewegt. Halterung und Antrieb der Lanzettennadel und Fixierung des Lanzettenkörpers können, wie oben beschrieben, durch geeignete konstruktive Maßnahmen in der Stechhilfe realisiert werden.

Das elastische Material, welches einen Teil des hohlen Lanzettenkörpers ausmacht, wird beim Stechvorgang von der Lanzettennadelspitze durchstoßen und verschließt sich gegebenenfalls nach dem Zurückziehen der Lanzettennadelspitze in den Hohlkörper wieder und dichtet so den Hohlkörper ab. Die Lanzettennadelspitze ist somit bis unmittelbar vor Gebrauch steril im Hohlkörper versiegelt und wird nach Gebrauch hygienisch in ihm eingeschlossen.

Die Lanzette dieser Ausführungsform kann ebenso wie die Lanzette des oben beschriebenen alternativen Erfindungsgegenstands neben dem Lanzettenkörper, der die Spitze der Lanzettennadel umschließt, einen weiteren Lanzettenkörper aufweisen, der im Zusammenspiel mit geeigneten Elementen einer Stechhilfe während des Stechvorgangs mit diesen wechselwirkt. Ebenso kann die Lanzettennadel speziell geformt sein, beispielsweise einen Kopf an dem der Spitze entgegengesetzten Ende besitzen.

Für die Eigenschaften des elastischen Materials und die Verbindung des elastischen Material mit dem steifen Material des Lanzettenkörpers gilt entsprechend das oben bezüglich der ersten Ausführunsgform Gesagte.

Der Bluttransfer von der Wunde/Einstichstelle der Lanzette zum Messort wird erfindungsgemäß auf zwei prinzipiel zu unterscheidende Arten realisiert: Zum einen kann der Bediener des analytischen Hilfsmittels nach dem Stechvorgang manuell den gewonnenen Blutstropfen auf das entsprechende Testelement übertragen. Vorzugsweise wird der Bluttransfer jedoch ohne Zutun des Benutzers des erfindungsgemäßen Dispos, quasi "automatisch" bewerkstelligt. Zu diesem Zweck kann das Dispo Mittel zum Probenflüssigkeitstransport aufweisen. Vorzugsweise sind diese Mittel kapillaraktiv, beispielsweise als Spalte oder Kanäle in einem starren Grundkörper oder als saugfähige Matrixmaterialien ausgebildet. Möglich ist auch eine Kombination dieser beiden prinzipiellen Möglichkeiten, beispielsweise dass das Blut zunächst über einen Kapillarkanal geführt, von einem saugfähigen Matrixmaterial übernommen und an ein Testelement abgegeben wird.

Als saugfähige Matrixmaterialien im Sinne dieser Erfindung haben sich insbesondere saugfähige Vliese, Papiere, Dochte, oder Gewebe als geeignet erwiesen.

In einer bevorzugten Variante enthält der Grundköper (Lanzettenkörper) des analytischen Hilfsmittels das Mittel zum Probenflüssigkeitstransport. Dies kann ein saugfähiger, in den Lanzettenkörper eingelassener Docht oder - was bevorzugt ist - ein ausgeformten Kapillarspalt sein, der dicht neben der Nadel lokalisiert ist und seinen Einlass in unmittelbarer Nähe der Nadelaustrittsöffnung hat. Dadurch wird erreicht, dass das Dispo zur Blutaufnahme nicht (oder nur minimal) lateral verschoben werden muss. Die Geometrie der Einlassöffnung ist derart gestaltet, dass sich der gebildete Blutstropfen möglichst leicht dort anlagern lässt, beispielsweise als Trichter oder Kerbe. Die Kapillarwirkung sorgt dann für das Ansaugen der benötigten Blutmenge, die deutlich unterhalb von 1 Mikroliter liegen kann. Das Blut gelangt auf diesem Wege zum Testfeld und reagiert dort mit der Testchemie, wobei ein auswertbares elektrisches Signal oder eine Farbänderung erzeugt wird. Der Kapillarspalt kann beim Spritzgießen in den Kunststoff eingeformt werden oder nachträglich in den Kunststoffkörper eingebracht werden, beispielsweise geprägt oder gefräst.

In einer weiteren bevorzugten Variante wird das Mittel zum Probenflüssigkeitstransport (z. B. ein Kapillarspalt oder ein Docht) nicht in den Kunststoff eingeformt, der den Lanzettenkörper bildet, sondern durch den spezifischen Aufbau des Testelementes erzeugt. Beispielsweise kann das Testelement analog zu WO 99/29429 oder EP-A 0 359 831 aufgebaut sein.

Zur sensorischen Erfassung von des Analyten, insbesondere von Blutglukose werden die im Stand der Technik bekannten Verfahren eingesetzt. Hierbei werden photometrische bzw. elektrochemische Verfahren bevorzugt.

Das analytische Testelement des erfindungsgemäßen analytischen Hilfsmittels kann aus einem Nachweisfilm bestehen, welcher direkt mit dem Lanzettenkörper verbunden ist. Solche Nachweisfilme sind in vielfältigen Ausführungsformen aus dem Stand der Technik bekannt. Beispielsweise wird in WO 99/29429 ein solcher Nachweisfilm beschrieben. Weiterhin kann - wie bereits oben erwähnt - ein komplettes herkömmliches Testelement mit dem Grundkörper/Lanzettenkörper des erfindungsgemäßen Hilfsmittels verbunden sein. Auch solche Testelemente sind dem Fachmann bekannt.

Die Verbindung des Testelements mit dem Lanzettenkörper kann auf vielfältige Weise geschehen. Ohne abschließend sein zu können seien erwähnt: Verkleben, Verschweißen, Verklipsen, Befestigung via Klettvertschlüssen oder Magneten, Vernähen, Verschrauben und dergleichen mehr. Bevorzugt wird das Testelement mit dem Lanzettenkörper verklebt, beispielsweise mittels Schmelzkleber oder mittels einem beidseitig klebenden Klebeband.

Allgemein lässt sich die Funktion des erfindungsgemäßen Disposables wie folgt schildern:
1. Das Dispo wir in die Aufnahmevorrichtung eines (Blutzucker-) Messgerätes gesteckt und damit fixiert.
2. Der Antriebsmechanismus der Stecheinheit wird gespannt und koppelt an die Nadelverdickung des Dispos an
3. Beim Auslösen des Stechvorgangs wird die Nadel nach vorne bewegt und tritt dabei aus dem Weichkunststoff mit hoher Geschwindigkeit aus. Der gesamte Stechvorgang spielt sich im Bereich weniger Millisekunden ab.
4. Nach erfolgtem Einstich in die Haut wird die Nadel wieder in den Weichkunststoff zurückgezogen (Ausgangslage). Der Antrieb wird gegebenenfalls wieder abgekoppelt.
5. Nach Ausbildung eines geeigneten Blutstropfens wird die gesamte Aufnahmevorrichtung so weit vorgefahren, dass die Ansaugöffnung (z. B. Kapillare) den Tropfen kontaktiert. Alternativ ist es möglich, den Blutstropfen manuell auf die entsprechende Probenaufgabezone des Testelements aufzubringen.
6. Bei den Varianten des Disposables, die über Mittel zum Probenflüssigkeitstransport verfügen, wird durch die Saugwirkung dieser Mittel das Blut im Dispo an eine Stelle transportiert, wo mittels photometrischer oder elektrochemischer Reaktion ein Signal in Abhängigkeit von der Konzentration eines Blutbestandteils erzeugt wird.

Die Herstellung eines erfindungsgemäßen Disposables ist prinzipiell in den folgenden einfachen Schritten realisierbar:
(1) Spritzgießen des Grundköpers inklusive Einbettung der Lanzettennadel (ggf. mit Erzeugung des "Nadelkopfes", d. h. der von einem Stechgerät greifbaren Verdickung)
(2) Umspritzung der Nadelspitze mit Weichkunststoff
(3) Sterilisation der "Rohdispos" (die im wesentlichen aus der Lanzette, gegebenenfalls mit einem Kapillarkanal im Lanzettenkörper, besteht), beispielsweise mittels Gamma-Strahlung
(4) Testmontage, d. h. Verbinden des Testelements mit dem Grundkörper

Vorzugsweise können sowohl die "Rohdispos" als auch die Testelemente als Bandware vorliegen, die nach der Testmontage in Einzeldispos aufgeteilt wird, beispielsweise duch Schneiden oder Stanzen. Wichtig ist - unabhängig davon, ob die erfindungsgemäßen Disposables als Rollen- oder Bandware in einem kontinuierlichen Prozess oder batchweise oder einzeln gefertigt werden - dass Lanzette und Testelement erst miteinander verbunden werden, nachdem die Sterilisierung der Lanzettennadel stattgefunden hat. Eine Sterilisierung des fertig montierten Dispos hätte zur Folge, dass unter Umständen empfindliche chemische oder biologische Substanzen des Testfeldes geschädigt werden. Dies kann durch das beschriebene Verfahren vermieden werden.

Die Sterilität der Lanzettennadelspitze im unbenutzten Zustand ist durch geeignete Maßnahmen, wie beispielsweise Gamma-Bestrahlung, herzustellen. Einmal sterilisiert bleiben die Lanzettennadelspitzen durch die entsprechenden Lanzettenkörper, die unter anderem ein elastisches Material beinhalten, erhalten. Anders als im Stand der Technik, wo bislang keine elastischen Materialien zur Abschirmung von Lanzettennadelspitzen beschrieben sind, ermöglicht die Verwendung des elastischen Materials gemäß der vorliegenden Erfindung jedoch auch die hygienische Abschirmung der benutzten Lanzettennadelspitze. Durch die Verwendung des elastischen Materials wird ein eventuell kurzzeitig vorhandener Kanal, durch den die Lanzettennadel zum Zwecke des Stechens hindurchdringen kann, nach dem Zurückziehen der Lanzettennadel, das heißt nach vollzogenem Stechvorgang, wieder verschlossen. Eventuell nach dem Stechvorgang an der Lanzettennadelspitze anhaftende Kontaminationen, insbesondere Keime und infektiöses Material, können somit nicht oder nur stark reduziert an die Außenwelt gelangen. Dies ist von besonderem Vorteil für Einweglanzetten, die einzeln nach ihrer Benutzung entsorgt werden. Von ganz hervorragender Bedeutung ist diese Eigen schaft jedoch für Lanzettensätze und Lanzettenmagazine, bei denen neben unbenutzten auch benutzte Lanzetten gelagert werden, und die dann als Ganzes entsorgt werden können.

Die Erfindung weist die folgenden Vorteile auf:
- Die Spitze der Lanzettennadel ist bei allen Ausführungsformen im unbenutzten Zustand keimdicht abgeschirmt, das heißt, Keime können bis unmittelbar vor Benutzung der Lanzette nicht an die Lanzettennadelspitze vordringen. Nach geeigneter Sterilisierung bleiben die Lanzettenspitzen über lange Zeit steril.
- Die Sterilität der Lanzettennadel wird auch bei nachfolgenden Fertigungsschritten, wie zum Beispiel dem Verbinden von Lanzette und Testelement, gewährleistet. Dabei ist die empfindliche Nadelspitze vor mechanischern Einflüssen (Verbiegung etc.) geschützt.
- Die Spitze der Lanzettennadel kann bei allen Ausführungsformen im benutzten Zustand hygienisch abgeschirmt werden. Eine unbeabsichtigte Kontamination der Umgebung (Benutzer, Gegenstände, weitere Lanzetten) wird weitgehend ausgeschlossen.
- Der Benutzer der erfindungsgemäßen Dispos ist vor unbeabsichtigter Verletzung an einer benutzten Lanzettennadel geschützt. Gleiches gilt natürlich für andere Personen als den eigentlichen Benutzer.
- Die erfindungsgemäßen Dispos sind kostengünstig in großen Stückzahlen mit herkömmlichen Spritzgußverfahren herzustellen.
- Die erfindungsgemäßen Dispos sind weitgehend miniaturisierbar und eignen sich deshalb für den Einsatz in kompakten, automatisierten Systemen.
- Als analytische Testelemente können prinzipiell sämtliche bislang bekannten Varianten von Teststreifen oder Sensoren eingesetzt werden.

Die Erfindung wird durch die nachfolgenden Figuren näher erläutert:
Figur 1 zeigt schematisch in mehreren Ansichten eine erste bevorzugte Ausführungsform des erfindungsgemäßen analytischen Hilfsmittels.
Figur 2 zeigt schematisch in mehreren Ansichten eine zweite bevorzugte Ausführungsform des erfindungsgemäßen analytischen Hilfsmittels.
Figur 3 zeigt schematisch in mehreren Ansichten eine dritte bevorzugte Ausführungsform des erfindungsgemäßen analytischen Hilfsmittels.
Figur 4 zeigt schematisch in mehreren Ansichten eine vierte bevorzugte Ausführungsform des erfindungsgemäßen analytischen Hilfsmittels.
Figur 5 zeigt schematisch in mehreren Ansichten eine fünfte bevorzugte Ausführungsform des erfindungsgemäßen analytischen Hilfsmittels.
Figur 6 zeigt schematisch die Fertigung von analytischen Hilfsmitteln gemäß Figur 2 aus Bandware.

Obwohl in den einzelnen Figuren jeweils nur optisch auszuwertende Testelemente gezeigt sind soll dies nicht beschränkend für den Gegenstand der vorliegenden Erfindung sein. Vielmehr ist für den Fachmann klar, dass die Nachweisreaktion im Testelement mittels jeder beliebigen Methode verfolgt werden kann. Neben optischen Verfahren (wie beispielsweise Reflexionsphotometrie, Absorptionsmessung oder Fluoreszenzmessung) sind insbesondere elektrochemische Verfahren bevorzugt (wie zum Beispiel Potentiometrie, Amperometrie, Voltammetrie, Coulometrie).

Die Ziffern und Buchstaben in den Figuren bedeuten:
- 1: Analytisches Hilfsmittel (Disposable, Dispo)
- 2: Lanzette
- 3: Lanzettennadel
- 4: Lanzettenkörper
- 5: Hartkunststoffteil des Lanzettenkörpers
- 6: Teil des Lanzettenkörpers aus elastischem Material
- 7: Kapillarspalt
- 8: Verdickung am Nadelende
- 9: Testelement
- 10: Testfeld
- 11: Verschlussfolie
- 12: Verdickung in der Nadelmitte
- 13: Lanzettenband/-gurt
- 14: Testelementeband/-gurt

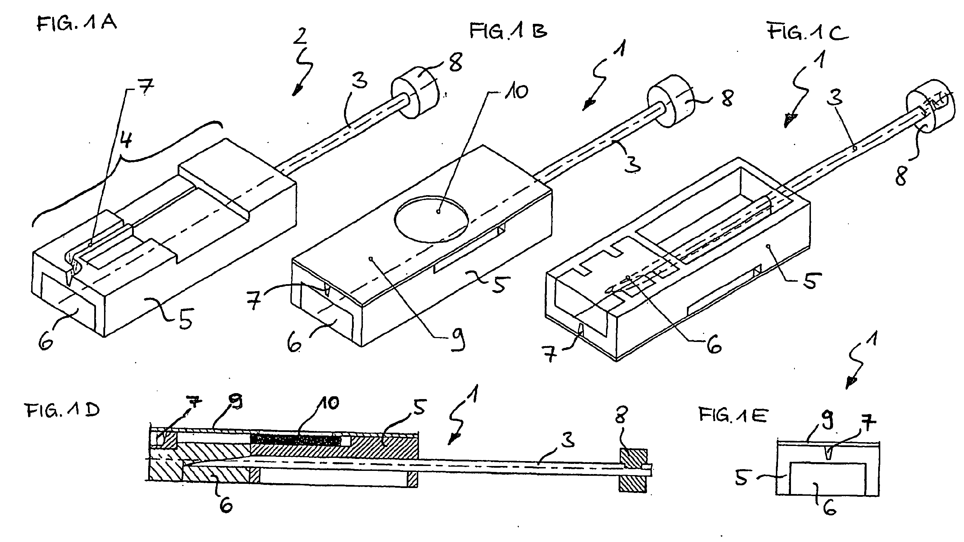

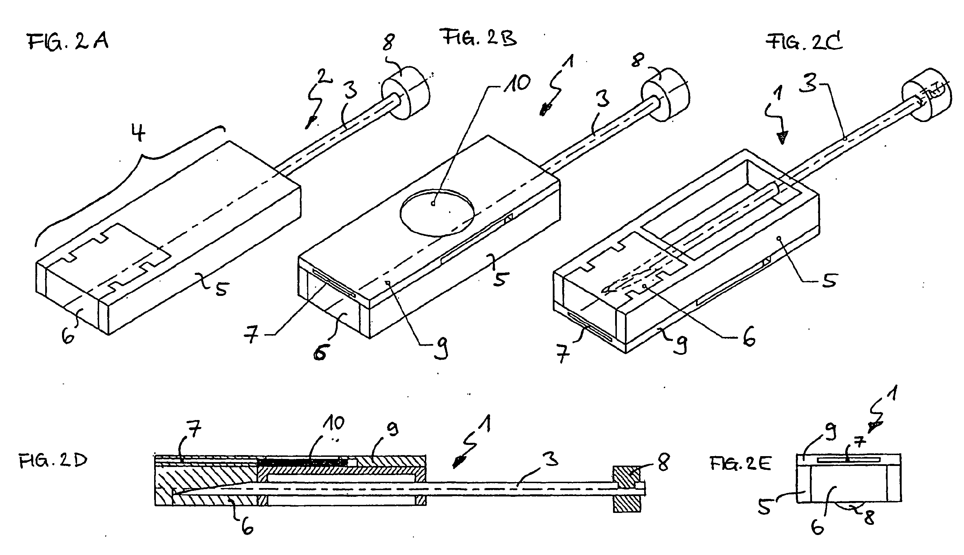

In Figur 1 ist schematisch in mehreren Teilfiguren (1A - 1E) eine bevorzugte Ausführungsform des analytischen Hilfsmittels abgebildet.

In Figur 1A ist zunächst die Lanzette (2) gezeigt. Diese enthält eine Lanzettennadel (3), die in einen Lanzettenkörper (4) eingebettet ist. Der Lanzettenkörper (4) besteht dabei aus einem Hartkunststoffteil (5) und einem Teil aus elastischem Material (6). Im Hartkunststoffteil (5) des Lanzettenkörpers (4) ist ein Kapillarspalt (7) eingearbeitet, der dem Probenflüssigkeitstransport dient.

Am hinteren Ende der Lanzettennadel (3) ist eine Verdickung (8) angebracht, die dazu dient, dass die Lanzettennadel in der Stechhilfe bzw. im Stechgerät leicht gegriffen werden kann.

In Figur 1B ist das fertige analytische Hilfsmittel (1) abgebildet. Auf dem Hartkunststoffteil (5) des Lanzettenkörpers (4) ist ein streifenförmiges Testelement (9) aufgebracht, welches ein Testfeld (10) enthält. Dieses ist über den Kapillarspalt (7) für die Probenflüssigkeit zugänglich.

In Figur 1C ist das analytische Hilfsmittel (1) perspektivisch von der Unterseite abgebildet. In dieser Darstellung wird deutlich, dass das Hartkunststoffteil (5) des Lanzettenkörpers (4) die Lanzettennadel (3) lediglich im Bereich zweier Stege berührt und hält. Weiterhin ist durch die gestrichelten Linien angedeutet, wie die Spitze der Lanzettennadel (3) im elastischen Material (6) des Lanzettenkörpers eingebettet ist.

In Figur 1D ist ein Längsschnitt durch das analytische Hilfsmittel (1) abgebildet.

In Figur 1E ist eine Frontansicht des analytischen Hilfsmittels (1) zu sehen.

In Figur 2 ist in mehreren Teilfiguren (2A - 2E) eine weitere bevorzugte Ausführungsform des erfindungsgemäßen analytischen Hilfsmittels abgebildet.

In Figur 2A ist zunächst die Lanzette (2) des analytischen Hilfsmittels (1) perspektivisch von oben zu sehen. Die Lanzette (2) besteht aus einer Lanzettennadel (3), die in einen Lanzettenkörper (4) eingebettet ist. Dieser besteht aus einem Hartkunststoffteil (5) und einem Teil aus elastischem Material (6). Zudem ist um das hintere Ende der Lanzettennadel (3) eine Verdickung (8) angebracht, die zum Greifen der Lanzettennadel (3) durch ein Stechinstrument dient.

Anders als in Figur 1 enthält das analytische Hilfsmittel in Figur (2) einen Kapillarspalt nicht im Lanzettenkörper (4), sondern als Teil des Testelements (9).

In Figur 2B ist das fertig montierte analytische Hilfsmittel (1) abgebildet, bei dem auf dem Lanzettenkörper (4) ein Testelement (9) befestigt ist. Dieses Testelement enthält einen Kapillarspalt (7), der das Testfeld (10) für eine Blutprobe zugänglich macht.

In Figur 2C ist eine perspektivische Darstellung des analytischen Hilfsmittels (1) gemäß Figur 2B von unten abgebildet. Wie in Figur 1C wird in Figur 2C deutlich, dass die Lanzettennadel (3) nur durch Stege mit dem Hartkunststoffteil (5) des Lanzettenkörpers (4) verbunden ist. Die Nadelspitze der Lanzettennadel (3) befindet sich vollständig eingebettet im elastischen Material (6) des Lanzettenkörpers.

Figur 2D stellt einen Längsschnitt des analytischen Hilfsmittels (1) gemäß Figur 2B dar. In Figur 2E ist eine entsprechende Frontalansicht des analytischen Hilfsmittels (1) gemäß Figur 2B zu sehen. Die Figuren 2D und 2E machen deutlich, dass der Kapillarspalt (7) Teil des Testelements (9) ist.

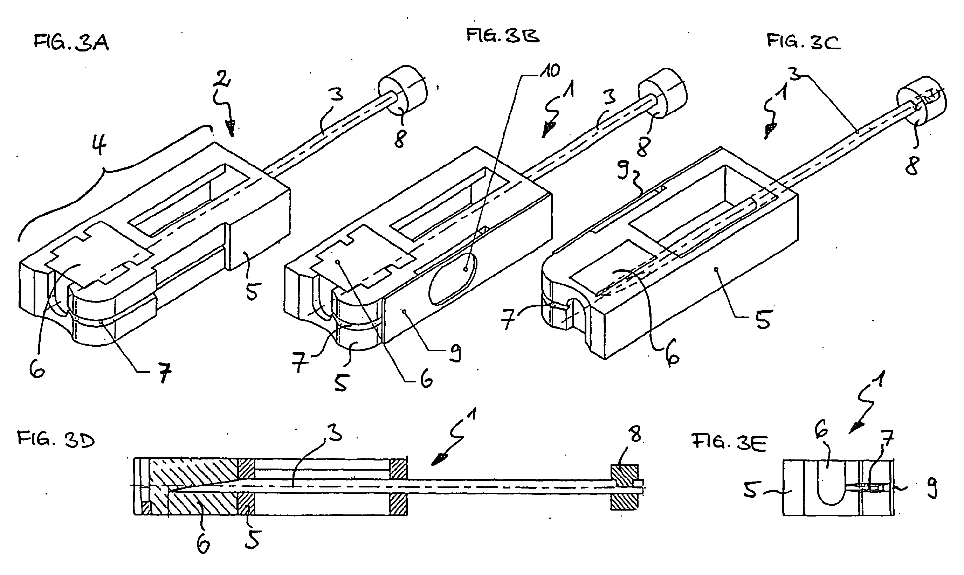

In Figur 3 ist in mehreren Teilfiguren (3A - 3E) eine weitere bevorzugte Ausführungsform des analytischen Hilfsmittels (1) der Erfindung dargestellt.

Ähnlich wie bei der Ausführungsform gemäß Figur 1 enthält die Ausführungsform gemäß Figur 3 einen Kapillarspalt (7) als Teil des Hartkunststoffteils (5) des Lanzettenkörpers (4). Lediglich die Lage des Kapillarspaltes (7) und die Lage des Testelements (9) unterscheiden sich von der der Ausführungsform gemäß Figur 1: Während bei der Ausführungsform gemäß Figur 1 Testelement (9) und Kapillarspalt (7) auf einer der großen Begrenzungsflächen des Lanzettenkörpers (4) angeordnet sind, sind diese Elemente in der Ausführungsform gemäß Figur 3 seitlich an einer der schmalen Begrenzungsflächen des Lanzettenkörpers (7) angeordnet. Funktion und Aufbau der Ausführungsform gemäß Figur 3 entspricht ansonsten im Wesentlichen dem, was für die Ausführungsform gemäß Figur 1 beschrieben wurde. Die Figuren 3A bis 3E entsprechen dabei den Figuren 1A bis 1E.

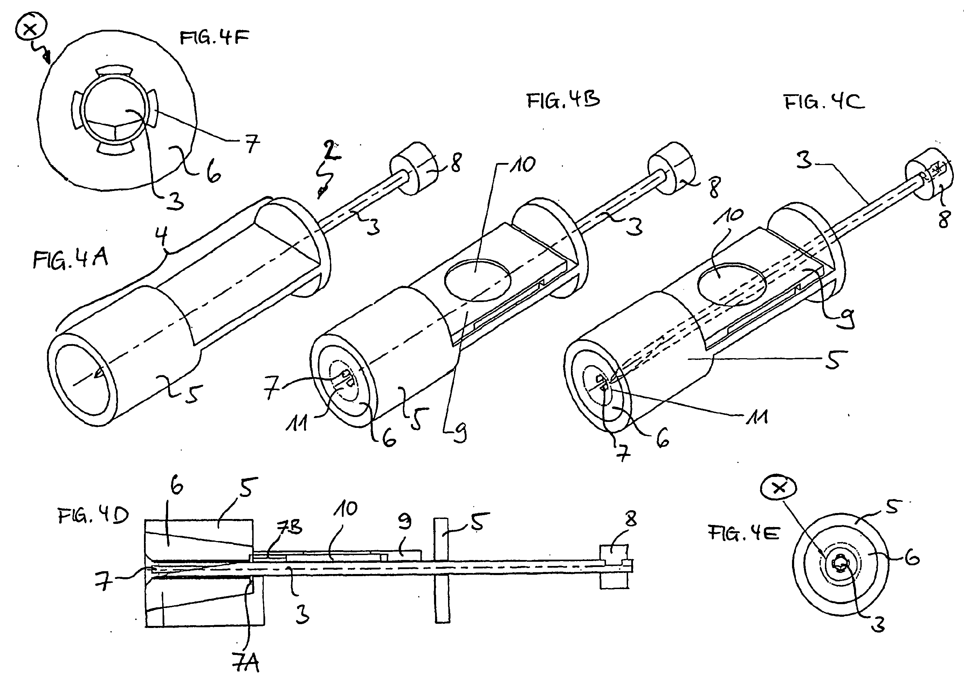

In Figur 4 ist in mehreren Teilfiguren (Figur 4A - 4F) eine weitere bevorzugte Ausführungsform des erfindungsgemäßen analytischen Hilfsmittels (1) abgebildet. Während bei den Ausführungsformen gemäß Figur 1 bis Figur 3 der Kapillarspalt (7) entweder Teil des Hartkunststoffteils (5) des Lanzettenkörpers (4) oder Teil des Testelements (9) war, ist bei der Ausführungsform gemäß Figur 4 der Kapillarspalt (7) zum Teil im elastischen Material (6) des Lanzettenkörpers (4) und zum Teil im Testelement (9) untergebracht. Wie insbesondere aus Figur 4D hervorgeht, lässt sich der Kapillarspalt (7) in drei Teilbereiche (7, 7A und 7B) unterteilen. Diese stehen miteinander in einem Kontakt, der einen Probenflüssigkeitstransport ermöglicht.

Wie bei den Ausführungsformen gemäß Figur 1 bis 3, besteht die Ausführungsform gemäß Figur 4 aus einer Lanzette (2), die eine Lanzettennadel (3) enthält, welche von einem Lanzettenkörper (4) teilweise umgeben ist. Der Lanzettenkörper (4) besteht dabei aus einem Hartkunststoffteil (5) und einem elastischen Material (6), welches insbesondere die Lanzettennadelspitze umgibt (vgl. Figur 4A). Am hinteren Ende der Lanzettennadel (3) ist eine Verdickung (8) an der Lanzettennadel angebracht, die wiederum das Greifen der Nadel (3) durch eine Stechhilfe ermöglichen soll.

Wie aus Figur 4B und 4C ersichtlich ist, ist auf den Lanzettenkörper (4) ein Testelement (9) aufgebracht, welches ein Testfeld (10) enthält. Wie bereits dargelegt, steht das Testfeld (10) über ein System von Kapillarkanälen (7, 7A, 7B) für Probenflüssigkeit zur Verfügung.

Die Austrittsöffnung der Lanzettennadel (3) ist in dieser Ausführungsform durch eine Verschlussfolie (11) verschlossen. Bei der Benutzung der Lanzette kann entweder die Verschlussfolie (11) von der Lanzettennadel (3) durchstochen werden oder aber die Verschlussfolie (11) wird manuell vor der Benutzung entfernt.

Figur 4E zeigt eine Frontalaufsicht auf die Austrittsöffnung der Lanzette des analytischen Hilfsmittels (1).

Figur 4F zeigt in vergrößerter Ansicht das mit X gekennzeichnete Detail der Frontalansicht gemäß Figur 4E. Insbesondere ist in dieser Ansicht zu erkennen, dass im elastischen Material (6) des Lanzettenkörpers (4) 4 Kapillarkanäle (7) vorhanden sind, die einen Probentransport zum Testelement (9) ermöglichen.

Bei den Ausführungsformen gemäß Figur 1 bis Figur 4 ist im hinteren Bereich der Lanzettennadel (3) eine Verdickung (8) vorgesehen, die für die Stechbewegung von zentraler Bedeutung ist. Diese Verdickung (8) ist so ausgeformt, dass ein Stechantrieb daran angekoppelt werden kann. In diesem Fall übernimmt der Antrieb sowohl die Hin- als auch die Rückbewegung der Nadel (3). Alternativ ist auch eine Antriebskopplung denkbar, bei der ein Stößel die Hinbewegung übernimmt. Die Rückbewegung erfolgt dann über eine Feder, die bei der Hinbewegung zusammengedrückt wird und sich anschließend wieder entspannt. Die Verdickung (8) an der Nadel (3) ist hierbei als einer der Anlagepunkte für die Feder wichtig. Diese Feder kann entweder Bestandteil des Disposables oder Bestandteil des Geräts oder einer Kassette bzw. eines Magazins sein. Die Verdickung (8) kann zum Beispiel durch ein aufgestecktes Teil aus Kunststoff oder Metall realisiert werden. Auch ein mechanisches Verformen (Quetschen, Biegen) der Nadel (3) zur Erzeugung einer Verdickung (8) ist denkbar.

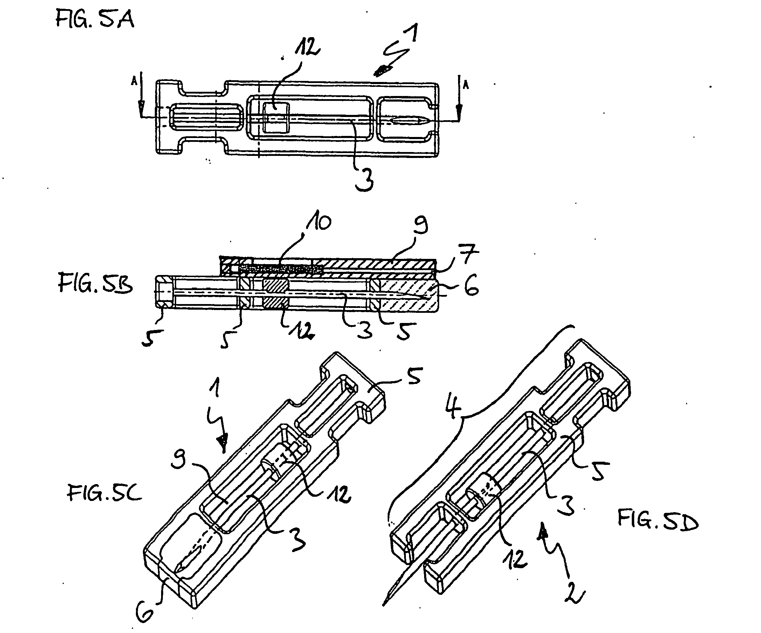

In Figur 5 ist in mehreren Teilfiguren (5A - 5D) eine weitere Ausführungsform der Erfindung zu sehen. In dieser Ausführungsform befindet sich die Verdickung (11) der Lanzettennadel (3) nicht an dem hinteren Ende der Lanzettennadel (3), sondern im Bereich der Nadelmitte. In diesem Fall ist die Verdickung im Inneren des Lanzettenkörpers (4) lokalisiert. Hier kann ein sinnvoller Antriebsmechanismus nur seitlich auf das Disposable zugreifen. Diese Lösung hat den Vorteil eines besonders kompakten und robusten Dispos.

Das analytische Hilfsmittel (1) entspricht ansonsten im Wesentlichen der Ausführungsform gemäß Figur 2.

Selbstverständlich ist es möglich, auch eine Ausführungsform gemäß Figur 1, d. h. eine Ausführungsform, bei der der Kapillarspalt (7) Teil des Lanzettenkörpers (4) ist, mit einer Verdickung (12) in der Nadelmitte zu realisieren.

In Figur 5A ist eine Ansicht auf das analytische Hilfsmittel (1) von unten zu sehen. Figur 5B stellt eine Schnittansicht durch die Längsachse des analytischen Hilfsmittels (1) dar. Figur 5C ist eine perspektivische Aufsicht auf die Unterseite des analytischen Hilfsmittels (1), bei dem die Lanzettennadel (3) sich in einer Position befindet, in der sie vor bzw. nach dem Stechvorgang ist. Die Spitze der Lanzettennadel ist dabei in das elastische Material (6) des Lanzettenkörpers eingebettet. In Figur 5D ist - zur Verdeutlichung ohne Testelement (9) und elastisches Material (6) - die Lanzette (2) in der Position abgebildet, in der die Lanzettennadel (3) während des Stechvorgangs vorliegt. Die Lanzettennadelspitze ragt dabei aus den Konturen des Lanzettenkörpers (4) heraus.

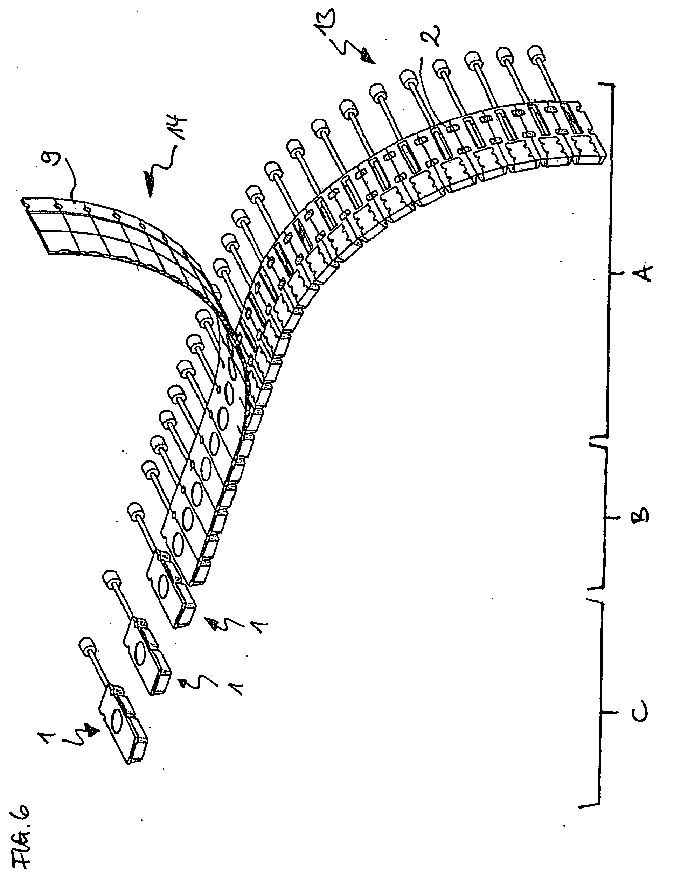

In Figur 6 ist stark vereinfacht schematisch wiedergegeben, wie die Fertigung von analytischen Hilfsmitteln (1) gemäß Figur 2 aus Bandmaterial von sich geht. Im Bereich A werden dabei zu einem Band bzw. Gurt (13) zusammengefasste Lanzetten (12) sowie zu einem Band bzw. Gurt (14) zusammengefasste Testelemente (9) bereitgestellt. Im Bereich B werden die beiden Bänder vereinigt und das Testelementband (14) mit dem Lanzettenband (13) verklebt. Im Bereich C schließlich erfolgt eine Vereinzelung der miteinander verbundenen Bänder zu einzelnen analytischen Hilfsmitteln (1), beispielsweise durch Abschneiden der endständigen Hilfsmittel (1).

## Patentansprüche

1. Analytisches Hilfsmittel (1), enthaltend
i) eine Lanzette (2), umfassend
eine Lanzettennadel (3) mit einer Spitze und
einen Lanzettenkörper (4), der die Lanzettennadel zumindest im Bereich der Spitze vollständig umgibt,
wobei die Lanzettennadel relativ zum Lanzettenkörper verschiebbar ist, und
wobei der Lanzettenkörper zumindest im Bereich der Spitze der Lanzettennadel aus
einem elastischen Material besteht, in das die Spitze der Lanzettennadel eingebettet ist, sowie
ii) ein analytisches Testelement (9),
wobei das analytische Testelement fest mit dem Lanzettenkörper verbunden ist

2. Analytisches Hilfsmittel (1), enthaltend
i) eine Lanzette (2), umfassend
eine Lanzettennadel (3) mit einer Spitze und
einen Lanzettenkörper (4), der im Bereich der Spitze der Lanzettennadel als Hohlkörper ausgebildet ist und der die Spitze der Lanzettennadel umgibt,
wobei die Lanzettennadel relativ zum Lanzettenkörper verschiebbar ist, und
wobei der Hohlkörper zumindest teilweise aus einem elastischen Material besteht, das von der Spitze der Lanzettennadel beim Stechvorgang durchstoßen werden kann und das sich gegebenenfalls nach dem Zurückziehen der Spitze der Lanzettennadel in den Hohlkörper wieder verschließt,
sowie
ii) ein analytisches Testelement (9),
wobei das analytische Testelement fest mit dem Lanzettenkörper verbunden ist.

3. Analytisches Hilfsmittel gemäß Anspruch 1 oder 2, **dadurch gekennzeichnet, dass** das elastische Material ein thermoplastisches Elastomer ist.

4. Analytisches Hilfsmittel gemäß Anspruch 1, 2 oder 3, **dadurch gekennzeichnet, dass** der Lanzettenkörper Mittel zum Probemlüssigkeitstransport aufweist.

5. Analytisches Hilfsmittel gemäß Anspruch 1, 2 oder 3, **dadurch gekennzeichnet, dass** das Testelement Mittel zum Probennüssigkeitstransport aufweist.

6. Analytisches Hilfsmittel gemäß Anspruch 4 oder 5, **dadurch gekennzeichnet, dass** das Mittel zum Probenflüssigkeitstransport ein Kapillarspalt, ein Kapillarkanal oder ein Docht oder Steg aus einem saugfähigen Material ist.

7. Analytisches Hilfsmittel gemäß Anspruch 4, 5 oder 6, **dadurch gekennzeichnet, dass** das Mittel zum Probenflüssigkeitstransport eine Probenaufgabestelle aufweist, die der Austrittsöffnung der Spitz der Lanzettennadel unmittelbar benachbart ist

## Claims

1. Analytical device (1) containing
i) a lancet (2) comprising
a lancet needle (3) with a tip and
a lancet body which completely surrounds the lancet needle at least in the area of the tip,
the lancet needle being movable relative to the lancet body and the lancet body being composed, at least in the area of the tip of the lancet needle, of an elastic material in which the tip of the lancet needle is embedded
and
ii) an analytical test element (9),
the analytical test element being permanently connected to the lancet body.

2. Analytical device (1) containing
i) a lancet (2) comprising
a lancet needle (3) with a tip and
a lancet body (4) which is in the form of a hollow body in the area of the tip of the lancet needle and which surrounds the tip of the lancet needle,
the lancet needle being movable relative to the lancet body and the hollow body being composed at least partially of an elastic material that can be pierced by the tip of the lancet needle during the lancing process and which optionally reseals after the tip of the lancet needle has been retracted into the hollow body
and
ii) an analytical test element (9)
the analytical test element being permanently connected to the lancet body.

3. Analytical device as claimed in claim 1 or 2, **characterized in that** the elastic material is a thermoplastic elastomer.

4. Analytical device as claimed in claim 1, 2 or 3, **characterized in that** the lancet body has means for sample liquid transport.

5. Analytical device as claimed in claim 1, 2 or 3, **characterized in that** the test element has means for sample liquid transport.

6. Analytical device as claimed in claim 4 or 5, **characterized in that** the means for sample liquid transport is a capillary gap, a capillary channel or a wick or a bar made of an absorbent material.

7. Analytical device as claimed in claim 4, 5 or 6, **characterized in that** the means for sample liquid transport has a sample application site which is directly adjacent to the outlet opening of the tip of the lancet needle.

## Revendications

1. Auxiliaire d'analyse (1) comprenant
i) une lancette (2) comprenant
une aiguille de lancette (3) munie d'une pointe et
un corps de lancette (4) qui entoure complètement l'aiguille de lancette, au moins dans la zone de la pointe,
l'aiguille de lancette étant déplaçable par rapport au corps de lancette, et
le corps de lancette étant constitué, au moins dans la zone de la pointe de l'aiguille de lancette, d'un matériau élastique dans lequel est incorporée la pointe de l'aiguille de lancette,
et
ii) un élément de test analytique (9),
l'élément de test analytique étant fermement relié au corps de lancette.

2. Auxiliaire d'analyse (1) comprenant
i) une lancette (2) comprenant
une aiguille de lancette (3) munie d'une pointe et
un corps de lancette (4) qui, dans la zone de la pointe de l'aiguille de lancette, est constituée sous forme de corps creux et entoure la pointe de l'aiguille de lancette,
l'aiguille de lancette étant déplaçable par rapport au corps de lancette, et
le corps creux étant constitué, au moins en partie, d'un matériau élastique qui, lors du processus de piqûre, peut être traversé par la pointe de l'aiguille de lancette, et qui, après le retrait de la pointe de l'aiguille de lancette dans le corps creux, se referme le cas échéant,
et
ii) un élément de test analytique (9),
l'élément de test analytique étant fermement relié au corps de lancette.

3. Auxiliaire d'analyse selon la revendication 1 ou 2, **caractérisé en ce que** le matériau élastique est un élastomère thermoplastique.

4. Auxiliaire d'analyse selon la revendication 1, 2 ou 3, **caractérisé en ce que** le corps de lancette comporte un moyen destiné au transport d'échantillon liquide.

5. Auxiliaire d'analyse selon la revendication 1, 2 ou 3, **caractérisé en ce que** l'élément de test comporte un moyen destiné au transport d'échantillon liquide.

6. Auxiliaire d'analyse selon la revendication 4 ou 5, **caractérisé en ce que** le moyen destiné au transport d'échantillon liquide est une fente capillaire, un canal capillaire ou une mèche ou nervure en matériau absorbant.

7. Auxiliaire d'analyse selon la revendication 4, 5 ou 6, **caractérisé en ce que** le moyen destiné au transport d'échantillon liquide présente une zone de réception d'échantillon qui est disposée immédiatement à côté de l'orifice de sortie de la pointe de l'aiguille de lancette.
